**Europäisches Patentamt**

(19) **European Patent Office**

**Office européen des brevets**

(11) Veröffentlichungsnummer: **0 039 088**
**B1**

(12) ## EUROPÄISCHE PATENTSCHRIFT

(45) Veröffentlichungstag der Patentschrift:
01.02.84

(51) Int. Cl.³ **G 01 N 21/85,** G 01 N 33/18

(21) Anmeldenummer: 81103283.8

(22) Anmeldetag: 30.04.81

(54) Messanordnung zur Bestimmung der Konzentration gelöster organischer Stoffe in einer auch suspendierte Feststoffe enthaltenden Flüssigkeit.

(30) Priorität 30.04.80 JP 56254/80

(43) Veröffentlichungstag der Anmeldung:
04.11.81 Patentblatt 81/44

(45) Bekanntmachung des Hinweises auf die Patenterteilung:
01.02.84 Patentblatt 84/5

(84) Benannte Vertragsstaaten.
**DE FR GB IT**

(56) Entgegenhaltungen·
**DE - A - 1 598 306**
**DE - A - 2 160 836**
**DE - A - 2 233 949**
**DE - A - 2 518 857**
**US - A - 3 526 462**
**US - A - 4 077 724**

(73) Patentinhaber: **FUJI ELECTRIC CO. LTD., 1-1, Tanabeshinden, Kawasaki-ku, Kawasaki 210 (JP)**

(72) Erfinder: **Sukigara, Kunio, 272-6 Ogino Yokosuka-shi, Kanagawa (JP)**

(74) Vertreter: **Mehl, Ernst, Dipl.-Ing., Postfach 22 01 76, D-8000 München 22 (DE)**

EP 0 039 088 B1

### Meßanordnung zur Bestimmung der Konzentration gelöster organischer Stoffe in einer auch suspendierte Feststoffe enthaltenden Flüssigkeit

Die Erfindung bezieht sich auf eine Meßanordnung zur Bestimmung der Konzentration gelöster organischer Stoffe in einer auch suspendierte Feststoffe enthaltenden Flüssigkeit.

Bei der Überwachung von Flüssigkeiten, insbesondere von Abwasser, ist die Bestimmung einer Reihe von möglichen Verunreinigungen, insbesondere von gelösten organischen Substanzen, von besonderer Wichtigkeit.

Ein Meßverfahren nach der sogenannten Kalium-Permanganat-Methode befindet sich bereits im Einsatz. Es ist jedoch relativ aufwendig, bedarf häufiger Wartung und erlaubt keine kontinuierliche Messung. Andere bekannte Meßmethoden beruhen auf der Absorption von durch die Flüssigkeitsprobe gesandter Lichtstrahlung durch die darin gelösten organischen Stoffe. So ist beispielsweise in der US-A-4 077 724 eine Meßanordnung beschrieben, die aus einer im sichtbaren und ultravioletten Spektralbereich strahlenden Strahlungsquelle, einem die Probenflüssigkeit enthaltenden Meßgerät mit einander gegenüberliegenden, die Meßstrecke im Strahlengang der Strahlungsquelle begrenzenden strahlungsdurchlässigen Fenstern, Mitteln zur Aufspaltung der Strahlung in eine sichtbare und in eine ultraviolette Teilstrahlung, Mitteln durch Detektierung der Teilstrahlen nach Durchgang durch die Meßstrecke und einer Schaltungsanordnung zur Bildung von Quotienten aus den Detektorausgangssignalen besteht.

Es besteht deshalb die Aufgabe, eine Meßanordnung zur Bestimmung des Anteils an gelösten organischen Substanzen in Flüssigkeiten, insbesondere in Wasser, zu schaffen, bei der der störende Einfluß von suspendierten Feststoffen kompensiert wird.

Eine Lösung dieser Aufgabe wird durch eine Meßanordnung gelöst, welche die im Anspruch 1 angegebenen Merkmale aufweist.

Bei dieser Meßanordnung läßt sich aus vier aufeinanderfolgend gewonnenen Meßsignalen, entsprechend der exponentiellen Abhängigkeit der vom Detektor empfangenen Strahlung vom Absorptionskoeffizienten bei der Wellenlänge $\lambda 1$ bzw. der Wellenlänge $\lambda 2$ und von der Weglänge $l_1$ bzw. $l_2$ in der Probenflüssigkeit, durch mehrfache Quotientenbildung ein Ausgangssignal gewinnen, welches dem Anteil an gelösten organischen Stoffen in der Probenflüssigkeit proportional ist und nicht von dem Gehalt an suspendierten Stoffen der Probenflüssigkeit beeinflußt wird.

Darüber hinaus lassen sich die durch die Alterung der Strahlungsquelle und des optoelektronischen Detektors sowie durch Verschmutzungserscheinungen in den optisch wirksamen Teilen des Strahlengangs hervorgerufene Störeinflüsse wirksam unterdrücken, wie es bereits an sich aus der DE-A-2 160 836 bekannt ist. Dort wird eine Meßküvette mit abgestuftem Querschnitt verwendet zur Gewinnung zweier unterschiedlicher, optisch wirksamer Weglängen in dem die Probenflüssigkeit enthaltenden Strahlengang.

Bei einer bevorzugten praktischen Ausführungsform der erfindungsgemäßen Meßanordnung wird der strahlungsdurchlässige Verdrängungskörper in einer besonderen Stellung mit Hilfe von anliegenden Abstreifkörpern gereinigt, während der den Verdrängungskörper axial bewegende Kolben gleichzeitig die Ein- und Austrittsfenster des Meßgefäßes säubert.

Weitere Einzelheiten der erfindungsgemäßen Meßanordnung sind aus der schematischen Darstellung eines Ausführungsbeispiels in Fig. 1, einer in der Praxis eingesetzten Ausführung in Fig. 2 sowie der Darstellung eines Filterrads in Fig. 3 in Verbindung mit den Unteransprüchen und der Beschreibung zu entnehmen.

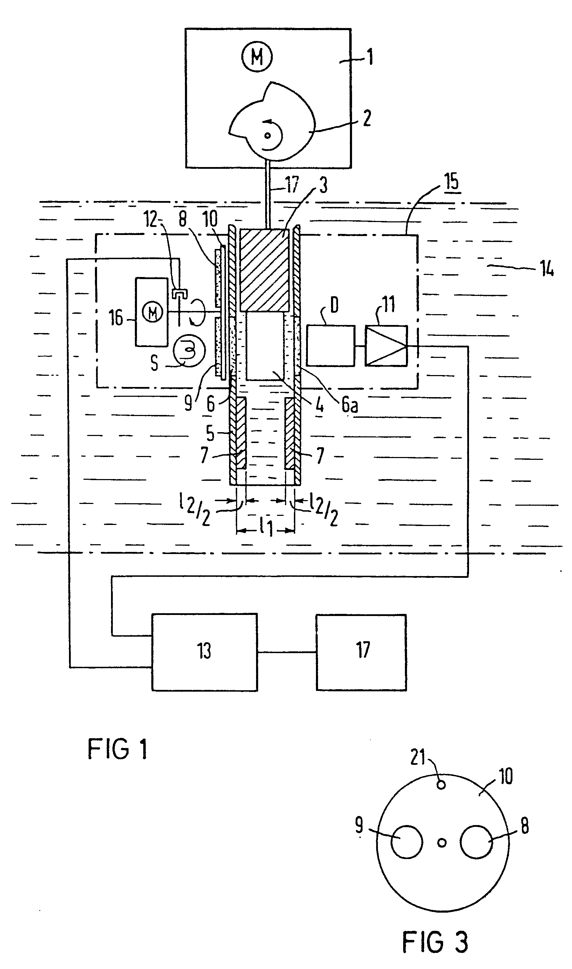

Fig. 1: Ein Meßwertaufnehmer 15 taucht in die Probenflüssigkeit 14, beispielsweise verunreinigtes Wasser, ein. Der Meßwertaufnehmer 15 enthält eine optische Strahlungsquelle S und eine Filtereinrichtung, die aus einer umlaufenden Filterscheibe 10 mit zwei symmetrisch zur Rotationsachse angeordneten Öffnungen, in welche Filter 8 und 9 eingesetzt sind, besteht. Zum Antrieb der Filterscheibe 10 dient der Motor 16. Das Filter 8 ist durchlässig in einem die sichtbare Strahlung umfassenden Spektralbereich mit der Schwerpunktwellenlänge $\lambda 1$ bei etwa 400 nm, das andere Filter 9 ist durchlässig in einem UV-Spektralbereich mit der Schwerpunktwellenlänge $\lambda 2$ bei etwa 254 nm. Zwischen der Filterscheibe 10 und einem nicht selektiven Strahlendetektor D ist ein rohrförmiges Meßgefäß 5 angeordnet mit einem der Länge $l_1$ entsprechenden Innendurchmesser, welches an beiden Enden offen ist und von der Probenflüssigkeit 14 frei durchströmt werden kann. Die eigentliche Meßstrecke wird von einander gegenüberliegenden, strahlungsdurchlässigen Fenstern 6 und 6a begrenzt, welche in der Wand des Meßgefäßes 5, im Strahlengang zwischen Strahlungsquelle S und Detektor D liegend, angeordnet sind. Die eine optisch wirksame Weglänge $l_1$ der in der Meßstrecke befindlichen Probenflüssigkeit wird durch den Innendurchmesser des rohrförmigen Meßgefäßes 5 bestimmt.

In den Strahlengang der Meßstrecke läßt sich ein axial in dem rohrförmigen Meßgefäß 5 verschiebbarer Verdrängungskörper 4, beispielsweise aus Quarz, einbringen, mit Hilfe dessen die optisch wirksame Weglänge $l_1$ in der Probenflüssigkeit in der Meßstrecke sich auf die Länge $l_2$ verkürzen läßt. Der Verdrängungskörper 4 ist an der Stirnfläche eines in dem rohrförmigen Meßgefäß 5 bewegbaren Kolbens 3 befestigt, der mit Hilfe eines Antriebs 1 und einer Steuerscheibe 2 in mindestens zwei verschiedene Stellungen verbracht werden kann, nämlich in eine erste Stellung, in welcher der strahlungsdurchlässige Verdrängungskörper 4 sich außerhalb des Strahlengangs in der Meßstrecke befindet, und in eine zweite Stellung, in der, wie gezeichnet, der Verdrängungskörper 4 die optisch wirksame Weglänge in der Meßstrecke von $l_1$ auf $l_2$ verkürzt.

Falls notwendig, läßt sich der Kolben 3 mit dem Verdrängungskörper 4 auch noch in eine unterhalb der zweiten Stellung liegende dritte Stellung verbringen, in welcher Reinigungsmittel in Form von Abstreifkörpern 7 an seinen Lichtdurchtrittsflächen anliegen und diese beim Hinein- und beim Hinausbewegen reinigen.

Die Ausgangssignale des Detektors D werden in dem Vorverstärker 11 verstärkt und einer Auswerteschaltung 13 zugeführt, in welcher aus den vier, als Funktionen der Absorption in der Probenflüssigkeit in den zwei unterschiedlichen Spektralbereichen bei zwei unterschiedlichen optischen Weglängen $l_1$ und $l_2$ anfallenden Detektorausgangssignalen der Gelöststoffanteil in der Probenflüssigkeit errechnet und in 17 angezeigt bzw. registriert wird. Zur Steuerung der Abfolge bei der Verarbeitung der verschiedenen Signale ist ein auf der Welle des Motors 16 sitzender Synchronisierabgriff 12, beispielsweise eine im Lichtweg eines opto-elektronischen Abgriffs rotierende Unterbrecherscheibe, vorgesehen.

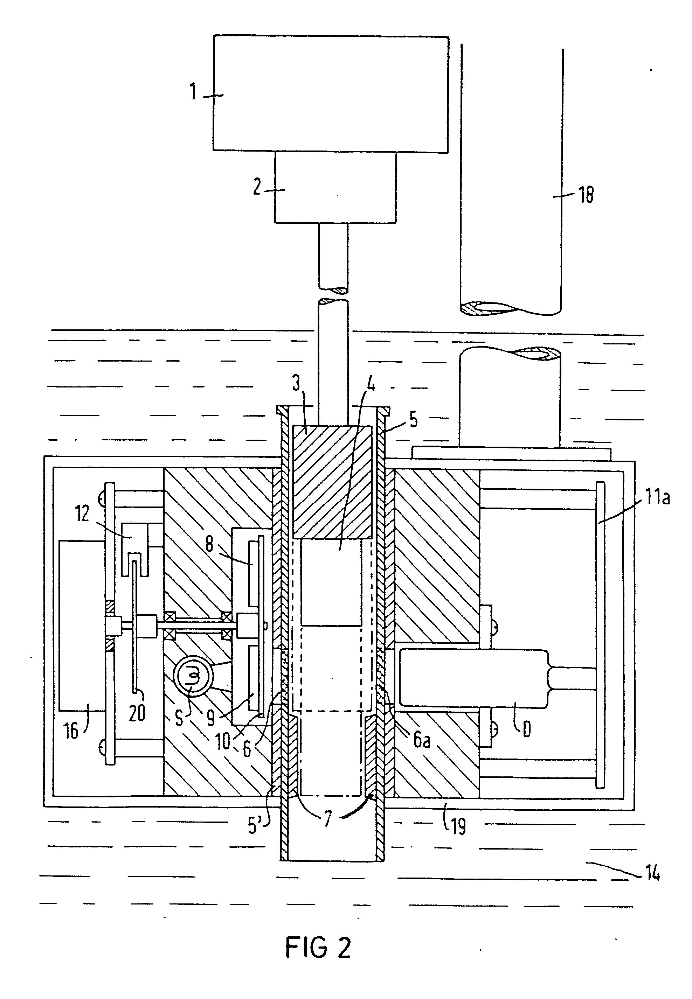

Fig. 2 zeigt die praktische Ausführung einer Meßanordnung nach Fig. 1. Gleiche Teile tragen gleiche Bezugszeichen.

Die Meßanordnung ist als Eintauchgeber ausgebildet, wobei die Strahlungsquelle S in Form einer Niederdruck-Quecksilberdampflampe, die von dem Motor 16 angetriebene Filterscheibe 10 mit den Filtern 8 und 9, das Meßgefäß 5 und der Detektor D mit dem Vorverstärker 11a in einem dichten Behälter 19 angeordnet sind und in die Probenflüssigkeit 14 eintauchen. Das eigentliche Meßgefäß 5 besteht aus einem Rohr aus strahlendurchlässigem Werkstoff, das in einer mit Strahlungsdurchtrittsöffnungen versehenen Hülse 5' gefaßt ist und dessen Enden aus dem dichten Behälter 19 in die Probenflüssigkeit 14 hineinragen, so daß diese ohne Behinderung in das Meßgefäß 5 ein- und austreten kann.

Der dichte Behälter 19 ist an einem hohlen Stiel 18 befestigt, durch welchen auch die elektrischen Zu- und Ableitungen für Strahlungsquelle S, den Motor 16 und den Detektor D geführt sind.

Die Strahlungsquelle S strahlt sowohl im sichtbaren wie im ultravioletten Spektralbereich. Ihre Strahlung fällt entweder durch das Filter 8 oder durch das Filter 9 und durch das Fenster 6 in das Meßgefäß 5, welches die Probenflüssigkeit 14 enthält und verläßt dieses durch das Fenster 6a. Die durch die Absorption in der Probenflüssigkeit geschwächte Strahlung erzeugt in dem Detektor D strahlungsproportionale Signale.

Mit Hilfe des Antriebs 1 und der Steuerscheibe 2, die oberhalb des Flüssigkeitsspiegels angeordnet und nur schematisch angedeutet sind,

kann der Kolben 3 mit dem daran befestigten Verdrängungskörper 4 in drei Stellungen gebracht werden.

Wie in der Zeichnung voll ausgezogen dargestellt, befindet sich der strahlendurchlässige Verdrängungskörper 4 in seiner oberen Stellung außerhalb des die Meßstrecke enthaltenden Strahlengangs zwischen der Strahlungsquelle S und dem Detektor D. In dieser Stellung wird einmal das Filter 8 und folgend das Filter 9 mit Hilfe der rotierenden Filterscheibe 10 in den Strahlengang gebracht, so daß bei gleicher optisch wirksamer Weglänge zwei Signale mit spektral abhängiger, unterschiedlicher Schwächung der Strahlung in der Meßstrecke auf den Detektor D gelangen.

Der Kolben 3 mit dem Verdrängungskörper 4 wird dann in die gestrichelt dargestellte zweite Stellung gebracht, in welcher der strahlendurchlässige Verdrängungskörper 4 sich im Strahlengang der Meßstrecke befindet und die optisch wirksame Weglänge in der Probenflüssigkeit um den Abstand zwischen seinen Lichtdurchtrittsflächen von $l_1$ auf $l_2$ verkürzt wird. In dieser zweiten Stellung des Verdrängungskörpers 4 werden wieder zwei Signale aus dem Detektor D gewonnen, die der Absorption der Probenflüssigkeit in einem Spektralbereich, entsprechend dem Durchlaßbereich des Filters 8 und folgend in einem entsprechend dem Durchlaßbereich des Filters 9 proportional sind.

Aus den vier Signalen wird — wie bereits beschrieben — die dem Gehalt an gelösten organischen Stoffen entsprechende Strahlungsabsorption bestimmt.

Der Kolben 3 mit dem strahlungsdurchlässigen Verdrängungskörper 4 läßt sich dann in eine strichpunktiert gezeichnete dritte Stellung verfahren, wobei der Verdrängungskörper 4 zwischen die Abstreifkörper 7 im unteren Teil des rohrförmigen Meßgefäßes 5 gebracht und dort beim Hinein- und Hinausführen gereinigt wird.

Die Innenflächen der Fenster 6 und 6a werden dabei gleichzeitig von der unteren Kante des Kolbens 3 überstrichen und gereinigt, während der Strahlengang durch den Kolben 3 unterbrochen wird. In dieser Stellung des Kolbens 3 kann der durch einfallendes Streulicht verursachte Störeinfluß mit Hilfe des Detektors D erfaßt und in der Auswerteschaltung 13 kompensiert werden.

In der Fig. 2 sind weiter noch eine den Vorverstärker 11a enthaltende bestückte Leiterplatte sowie der Synchronisierabgriff 12, der kapazitiver, induktiver, magnetischer oder opto-elektronischer Art sein kann, mit der in ihm umlaufenden Steuerscheibe 20 zu sehen.

In Fig. 3 ist ein Ausführungsbeispiel einer Filterscheibe 10 dargestellt, die zentrisch auf der rotierenden Welle des Motors 16 befestigt wird. Die Scheibe 10 aus strahlungsundurchlässigem Material ist mit zwei peripher und symmetrisch zur Rotationsachse angeordneten Öffnungen versehen, in welche die beiden optischen Filter 8 und 9 eingelassen sind.

Wird ein opto-elektronischer Abgriff 12 verwendet, so kann anstelle der Steuerscheibe 20 in Fig. 2 auch die Filterscheibe 10 eingesetzt werden. Die Scheibe 10 ist dann an ihrem, den Abgriff 12 durchlaufenden Rand mit mindestens einer Öffnung 21 versehen, die bei jeder Umdrehung der Scheibe 10 den Lichtweg des opto-elektronischen Abgriffs einmal freigibt und somit einen Steuerimpuls erzeugt.

**Patentansprüche**

1. Meßanordnung zur Bestimmung der Konzentration gelöster organischer Stoffe in einer auch suspendierte Feststoffe enthaltenden Flüssigkeit, insbesondere Abwasser, mit

a)  einer im sichtbaren und ultravioletten Spektralbereich strahlenden Strahlungsquelle (S),
b)  einem die Probenflüssigkeit (14) enthaltenden Meßgefäß (5) mit einander gegenüberliegenden, die Meßstrecke im Strahlengang der Strahlungsquelle (S) begrenzenden, strahlungsdurchlässigen Fenstern (6, 6a),
c)  ersten Mitteln zur Aufspaltung der Strahlung in eine sichtbare und eine ultraviolette Teilstrahlung,
d)  zweiten Mitteln zur Detektierung der Teilstrahlungen nach Durchgang durch die Meßstrecke,
e)  einer Schaltungsanordnung zur Bildung von Quotienten aus den Detektorausgangssignalen,

dadurch gekennzeichnet,

—  daß die ersten Mittel eine Filtereinrichtung (8, 9, 10) aufweisen, die abwechselnd die ultraviolette Teilstrahlung, nämlich die Strahlung eines Absorptionsbanden von suspendierten Feststoffen und gelösten organischen Stoffen enthaltenden ersten Spektralbereichs und die sichtbare Teilstrahlung, nämlich die Strahlung eines nur Absorptionsbanden von suspendierten Feststoffen enthaltenden zweiten Spektralbereichs, in die Meßstrecke eintreten läßt,
—  daß ein strahlungsdurchlässiger Verdrängungskörper (4) zur Verkürzung der wirksamen optischen Weglänge ($l_1$) in der Probenflüssigkeit periodisch für die Dauer mindestens eines Filterwechselzyklus in die Meßstrecke einführbar ist,
—  daß die zweiten Mittel einen nicht-selektiven Strahlungsdetektor (D) aufweisen, der nach dem Austrittsfenster (6a) der Meßstrecke angeordnet ist,
—  und daß die Auswerteschaltung aus einer von dem Strahlungsdetektor (D) gespeisten Auswerteschaltung (13) besteht, in welcher aus den vier als Funktionen der Absorption in den zwei unterschiedlichen Spektralbereichen bei zwei unterschiedlichen optischen Weglängen ($l_1$, $l_2$) anfallenden Detektorausgangssignalen der Anteil an gelösten organischen Stoffen in der Probenflüssigkeit errechnet und angezeigt ist.

2. Meßanordnung nach Anspruch 1, mit einer Quecksilberdampflampe als Strahlungsquelle (S), dadurch gekennzeichnet,

—  daß die Filtereinrichtung aus einer motorisch angetriebenen Filterscheibe (10) besteht, die zwei symmetrisch zur Rotationsachse angeordnete Filter (8, 9) aufweist, von denen eines in dem sichtbare Strahlung umfassenden Spektralbereich mit der Schwerpunktwellenlänge bei etwa 400 nm, das andere in einem UV-Spektralbereich mit der Schwerpunktwellenlänge bei etwa 254 nm durchlässig ist,
—  daß das Meßgefäß aus einem rohrförmigen Meßgefäß (5) besteht, in dessen Mantelfläche einander gegenüberliegende und mit den Fenstern (6, 6a) verschlossene, die Meßstrecke begrenzende Öffnungen, und in dessen Stirnflächen Eintritts- und Austrittsöffnungen für die Probenflüssigkeit (14) angebracht sind,
—  und daß der Verdrängungskörper aus einem Verdrängungskörper (4) aus Quarz besteht, der an einem im rohrförmigen Meßgefäß (5) axial in mindestens zwei Stellungen verschiebbaren Kolben (3) befestigt ist, wobei sich in der ersten Stellung der Verdrängungskörper (4) außerhalb, in der zweiten Stellung innerhalb der Meßstrecke befindet.

3. Meßanordnung nach Anspruch 1 oder 2, dadurch gekennzeichnet, daß Strahlungsquelle (S), Filtereinrichtung (8, 9, 10), Meßgefäß (5) und Detektor (D) in einem dichten Behälter (19) vereint in die Probenflüssigkeit (14) eintauchbar sind und daß das Meßgefäß (5), als beidseitig offene Röhre ausgebildet, mit seinen Enden aus dem Behälter (19) ragt.

4. Meßanordnung nach Anspruch 2, dadurch gekennzeichnet, daß der Verdrängungskörper (4) in eine dritte Stellung verbingbar ist, in welcher Reinigungsmittel in Form von Abstreifkörpern (7) an seinen Lichtdurchtrittsflächen anliegen.

**Claims**

1. A measuring arrangement for determining the concentration of dissolved organic substances in a liquid which also contains suspended solids, in particular sewage, comprising

—  a radiation source (S), radiating in the visible and ultra-violet spectral regions;
—  a measuring vessel (5) which contains the test liquid (14) and has radiation-transmissive windows (6, 6a) arranged opposite to one another which delimit the measuring section in the path of rays from the radiation source (S);

— first means for splitting the radiating into a visible and an ultra-violet radiation component;

— second means for detecting the radiation component after passage through the measuring path; and

— a circuit arrangement for the formation of quotients from the detector output signals,

characterised in

— that the first means has a filter device (8, 9, 10) which alternately allows the ultra-violet radiation component, namely the radiation of a first spectral region, which includes absorption bands of suspended solids and dissolved organic substances, and the visible radiation component, namely the radiation of a second spectral region, which only contains absorption bands of suspended solids, to enter the measuring path;

— that, in order to shorten the effective optical path length ($l_1$) in the test liquid, a radiation-transmissive displacement body (4) can be periodically inserted into the measuring path for the duration of at least one filter alternation cycle;

— that the second means has a non-selective radiation detector (D) which is arranged after the outlet window (6a) of the measuring path;

— and that the evaluation circuit consists of an evaluation circuit (13) which is fed by the radiation detector (D) and in which the proportion of dissolved organic substances in the test liquid is calculated from the four detector output signals which result as functions in the two different spectral regions with two different optical path lengths ($l_1$, $l_2$), and is indicated.

2. A measuring arrangement according to Claim 1, having a mercury-vapour lamp as a radiation source (S), characterised in

— that the filter device consists of a motordriven filter disc (10) having two filters (8, 9) which are symmetrically arranged with respect to the axis of rotation, one of which is transmissive in the spectral region comprising visible radiation where the mavelength of the point of main effectiveness is approximately 400 nm and the other filter is transmissive in an ultra-violet spectral region where the wavelength of the point of main effectiveness is approximately 254 nm;

— that the measuring vessel consists of a tubular measuring vessel (5), in the curved surface of which openings are arranged which are located opposite to one another and are closed by the windows (6, 6a) and bound the measuring path, and inlet and outlet openings for the test liquid (14) are arranged at the end faces of the measuring vessel (5);

— and that the displacement body consists of a displacement body (4) made of quartz and which is secured to a piston (3) which can be in at least two axial positions in the tubular measuring vessel (5), the displacement body (4) being outside the measuring path in the first position and within the measuring path in the second position.

3. A measuring arrangement according to Claim 1 or Claim 2, characterised in that the radiation source (S), the filter device (8, 9, 10), the measuring vessel (5) and the detector (D) in combination in a sealed container (19), can be immersed in the test liquid; and that the measuring vessel (5) is in the form of a tube which is open at both sides and which projects from the container (19) at its ends.

4. A measuring device according to Claim 2, characterised in that the displacement body (4) can be brought into a third position in which claensing means in the form of wiping bodies (7) rest against its surfaces through which light passes.

## Revendications

1. Appareil pour la détermination de la concentration de soluté organique dans un liquide contenant des matières solides en suspension, notamment dans de l'eau résiduaire, comprenant

— une source de rayonnement (5) rayonnant dans le domaine visible et ultraviolet du spectre,

— un récipient de mesure (5) contenant le liquide d'essai (14) et ayant des fenêtres (6, 6a) transparentes au rayonnement, opposées mutuellement et délimitant la section de mesure dans le trajet du faisceau de la source de rayonnement (S),

— des premiers moyens pour subdiviser le rayonnement en un rayonnement partiel visible et en un rayonnement partiel ultraviolet,

— des seconds moyens pour détecter le rayonnement partiel après traversée de la section de mesure,

— un montage pour former des quotients à partir des signaux de sortie du détecteur,

caractérisé en ce que:

— les premiers moyens comportent un dispositif à filtres (8, 9, 10) qui laisse pénétrer dans la section de mesure alternativement le rayonnement partiel ultraviolet, à savoir le rayonnement d'un premier domaine du spectre contenant des bandes d'absorption de matières solides en suspension et de solutés organiques, et le rayonnement partiel et visible, à savoir le rayonnement d'un second domaine du spectre ne contenant que des bandes d'absorption de matières solides en suspension,

— un corps plongeur (4) transparent au rayonnement peut être introduit dans la section de mesure périodiquement pour la durée d'au moins un cycle d'échange de filtres afin de raccourcir le chemin optique effectif ($l_1$), dans le liquide d'essai,

— les seconds moyens comportent un détecteur de rayonnement (D) non sélectif, qui est disposé en aval de la fenêtre de sortie (6a) de la section de mesure,

— et le circuit d'exploitation est constitué d'un circuit d'exploitation (13) alimenté par le détecteur de rayonnement (D) et dans lequel la proportion de solutés organiques dans le liquide d'essai est calculée à partir des quatre signaux de sortie du détecteur se produisant sous forme de fonctions de l'absorption dans les deux domaines différents du spectre pour deux chemins optiques de longueur différente ($l_1$, $l_2$) et est affichée.

2. Appareil suivant la revendication 1, comprenant une lampe à vapeur de mercure comme source de rayonnement (5), caractérisée en ce que

— le dispositif de filtrage est constitué d'un disque à filtres (10), qui comporte deux filtres (8, 9) disposés symétriquement par rapport à l'axe de rotation, dont l'un est transparent dans le domaine du spectre entourant le rayonnement visible ayant le centre de gravité spectral à 400 nm environ, et dont l'autre est transparent dans un domaine U.V. du spectre ayant le centre de gravité spectral à 254 nm environ,

— le récipient de mesure est constitué d'un récipient de mesure (5) tubulaire, dans la surface latérale duquel sont ménagées des ouvertures mutuellement opposées, fermées par les fenêtres (6, 6a) et délimitant la section de mesure et dans les surfaces frontales duquel sont ménagées des ouvertures d'entrée et de sortie pour le liquide d'essai (14),

— et le corps plongeur est constitué d'un plongeur (4) en quartz, qui est fixé à un piston (3) pouvant coulisser axialement dans le récipient de mesure (5) tubulaire en prenant au moins deux positions, le plongeur (4) se trouvant en la première position à l'intérieur et en la seconde position à l'extérieur de la section de mesure.

3. Appareil suivant la revendication 1 ou 2, caractérisé en ce que la source de rayonnement (S), le dispositif à filtres (8, 9, 10), le récipient de mesure (5) et le détecteur (D) réunis dans un récipient étanche (19) peuvent être plongés dans le liquide d'essai (14), et en ce que le récipient de mesure (5) est agencé en tuyau ouvert des deux côtés, dont les extrémités font saillie du récipient (19).

4. Appareil suivant la revendication 2, caractérisé en ce que le plongeur (4) peut être mis dans une troisième position dans laquelle des moyens de nettoyage sous forme de corps râcleurs (7) sont appliqués sur ses surfaces de passage de la lumière.

FIG 1

FIG 3

FIG 2